(19) Europäisches Patentamt

European Patent Office

Office européen des brevets

(11) Publication number: **0 041 330**
**A1**

(12) # EUROPEAN PATENT APPLICATION

(21) Application number: 81302164.9

(22) Date of filing: 15.05.81

(51) Int. Cl.³: **B 01 J 27/10**
**C 07 C 17/156, C 07 C 19/045**

(30) Priority: 19.05.80 US 151221

(43) Date of publication of application:
09.12.81 Bulletin 81/49

(84) Designated Contracting States:
BE DE FR GB IT NL

(71) Applicant: PPG INDUSTRIES, INC.
One Gateway Center
Pittsburgh Pennsylvania 15222(US)

(72) Inventor: Kearley, Robert Andrew
541 Chamberlain Street
Corpus Christi Texas 78408(US)

(74) Representative: Shipton, Gordon Owen et al,
W.P. THOMPSON & CO. Coopers Building Church Street
Liverpool L1 3AB(GB)

(54) Ethylene oxychlorination catalyst.

(57) A catalyst suitable for use in ethylene oxychlorination having cupric chloride and potassium chloride deposited on support material characterised in that substantially all of the cupric chloride and potassium chloride deposited on the support material is in the form of the trichlorocuprate complex $KCuCl_3$. A process for preparing ethylene dichloride using this catalyst and a process for preparing the catalyst is characterised in that a support material is treated with an aqueous solution consisting essentially of equimolar quantites of cupric chloride and potassium chloride and the treated support material is dried, the treating and drying steps being conducted at a temperature in excess of 76°C.

EP 0 041 330 A1

Croydon Printing Company Ltd.

- 1 -

DESCRIPTION

ETHYLENE OXYCHLORINATION CATALYST

The combination of cupric chloride and potassium chloride deposited on a support material is known to be effective in catalyzing the vapor phase oxychlorination of ethylene to produce ethylene dichloride. In this oxychlorination process, a gaseous mixture of ethylene, oxygen and a chlorine source, typically hydrogen chloride, is contacted with a fixed or fluidized bed of catalyst at moderately elevated temperature. The gaseous reaction stream containing ethylene dichloride and water vapor is condensed to form organic and aqueous liquid phases, the organic phase containing crude ethylene dichloride being worked-up in known fashion to produce purified ethylene dichloride product with the aqueous phase being discharged to waste.

Although conducting the ethylene oxychlorination reaction in the presence of a supported cupric chloride-potassium chloride catalyst results in satisfactorily high yields of ethylene dichloride, it has often been observed that the reaction is accompanied by an undesirably high rate of ethylene burning, i.e. an unduly high proportion of ethylene feedstock is burned to carbon dioxide rather than being converted to ethylene dichloride product.

It is believed that ethylene burning is attributable to a copper-rich catalyst resulting from uneven distribution of cupric and potassium chlorides on the support material.

In accordance with this invention, it has been found that, in the production of ethylene dichloride by vapour phase oxychlorination of ethylene in the presence of a catalyst comprising cupric chloride and potassium chloride carried on a particulate support material, the level of ethylene burning is reduced when substantially all of the cupric chloride and potassium chloride deposited on the support material are deposited in the form of the trichlorocuprate complex, $KCuCl_3$.

The catalyst, for use according to this invention, may be prepared by mixing or slurrying support material with an aqueous solution that is substantially equimolar in cupric chloride and potassium chloride followed by drying or evaporation of water, the temperature during both mixing or slurrying and drying or evaporation being maintained above $76^{\circ}C$.

In the ternary system $KCl-CuCl_2-H_2O$, two chlorocuprates are present, i.e. $K_2CuCl_4 \cdot 2H_2O$ and $KCuCl_3 \cdot 2H_2O$. Since in the aqueous solution used to treat the support material, cupric and potassium chlorides are present in substantially equimolar quantities, and since it is desired to deposit substantially all of the potassium and cupric chlorides on the support material in the form of the trichlorocuprate complex, $KCuCl_3$, it is necessary that the temperature during deposition and drying be maintained above $76^{\circ}C.$, for the $KCuCl_3$ complex cannot be deposited in substantially pure form at temperatures below $76^{\circ}C.$; but will be contaminated with greater or lesser amounts of the tetrachlorocuprate complex, $K_2CuCl_4$, and cupric chloride. Thus, in the preparation of catalyst for use in this invention, a uniform distribution of copper and potassium relative to each other can be assured by maintaining temperature above $76^{\circ}C.$ at all times during impregnation of the support material with the aqueous treating solution and subsequent drying of the impreganted support material.

As to choice of support material, the same may be any commonly used catalyst carrier, e.g., silica, silica gel, alumina, diatomaceous earth, and the like. If the catalyst is to be used in a fluidized rather than a fixed bed, silica or high silica (e.g. containing 40 to 55 percent $SiO_2$ and from 2 to 30 percent, typically 10 to 20 percent $Al_2O_3$) or alkali metal silicate content clay minerals are preferred, some examples of which are bentonite, kaolinite, illite and attapulgite clay minerals. Alumina or high alumina (e.g. containing in excess of 40 percent and typically in excess of 75 percent $Al_2O_3$) content clay minerals, e.g. diaspore and bauxite clays, may be used; however, these have been found to be somewhat more friable and to have a higher attrition rate when used in a fluidized bed than the predominantly silica or alkali metal silicate containing clays. Of the clay minerals, attapulgite is particularly preferred.

The quantity of trichlorocuprate complex $KCuCl_3$ deposited on the support material may vary over a wide range provided, of course, that substantially all of the cupric and potassium chlorides are present as the trichlorocuprate complex. Typically, sufficient trichlorocuprate complex is deposited on the support material so as to provide a copper content of from about 4 to 12 weight percent and preferably from about 7 to 9 weight percent based on the combined weight of trichlorocuprate complex and support material.

It is to be understood that the copper (and potassium) content of the catalyst is that resulting from depositing cupric and potassium chlorides on the support material and does not include copper and potassium that may be inherently present in the support material. It is to be further understood that the catalyst may also contain other known additives, such as for example, aluminium chloride as described in our U.S. Patent No. 4 172 052; in which case such other additives may be deposited on the support material prior to deposition of the trichlorocuprate complex in accordance with this invention.

In a typical practice of the invention, ethylene, hydrogen chloride and oxygen gases are fed, in known fashion, to a fixed or fluid bed reactor and, in the latter case, at a rate sufficient to maintain the catalyst bed in a fluidized condition without significant entrainment of catalyst particles in the product gas stream. The ratio of reactant gases, reaction temperature and contact time of reactant gases with the catalyst may vary but typically a mole ratio of ethylene:hydrogen chloride:oxygen of 1.0:1.5:0.5, a temperature of 150 to 500°C., usually 250 to 350°C., and a contact time of 10 seconds to about two minutes are usually employed. Particle size of the catalyst may also vary, and in the case of fluid bed operation, particle size of the catalyst is typically in the range of 30 to 200 mesh (U.S. Sieve), preferably between 40 and 100 mesh. Depending on reaction conditions, conversion of unburned ethylene to ethylene dichloride usually ranges from at least 70 percent to substantially quantitative and an ethylene dichloride crude having an ethylene dichloride content of from 97 to 99 percent or more may be obtained. Of course, the vapor phase oxychlorination of ethylene to ethylene dichloride may be conducted using a fixed rather than fluidized bed of catalyst in known manner and under known process conditions.

This invention may be further illustrated as follows:

(1) A quantity of an aqueous solution equimolar in potassium chloride and cupric chloride is mixed with attapulgite clay, sufficient of said solution being used so as to deposit on the clay, 8.0 ± 0.5 weight percent copper and 5.0 ± 0.5 weight percent potassium in the finished catalyst, the temperature during mixing and subsequent evaporation of water being maintained above 76°C. The catalyst, so prepared, is charged to an oxychlorination reactor wherein a gaseous stream of ethylene:hydrogen chloride:oxygen in a molar ratio of 1:1.5:0.45 respectively are introduced at a rate sufficient to maintain the catalyst in a fluidized state, the reaction temperature being maintained at from about 280°C to about 300°C, the contact time between the gaseous stream and catalyst being about 10 seconds. The extent of ethylene burning is observed to range from about 0.4 to about 0.7 percent basis ethylene in the feed gas.

(2) A quantity of aqueous solution equimolar in potassium chloride and cupric chloride is mixed with attapulgite clay sufficient of said solution being used so as to deposit from 8.0 ± 0.5 weight percent copper and 5.0 ± 0.5 weight percent potassium on the finished catalyst the temperature during mixing maintained at about 50°C and the mixture being dried at a temperature of 105°C. A quantity of this catalyst in amount corresponding to that prepared in (1) supra is charged to an oxychlorination reactor and a gaseous mixture of ethylene, hydrogen chloride and oxygen in the molar proportion, rate and at the reaction temperature specified in (1) supra is contacted with the catalyst. The extent of ethylene burning is observed to be about 2.4 percent basis ethylene in the feed gas.

- <u>6</u> -
<u>CLAIMS</u>

1. A catalyst suitable for use in ethylene oxychlorination having cupric chloride and potassium chloride deposited on support material characterised in that substantially all of the cupric chloride and potassium chloride deposited on the support material is in the form of the trichlorocuprate complex $KCuCl_3$.

2. A catalyst according to claim 1, characterised in that the copper content of the catalyst is from about 4 to about 12 weight percent.

3. A catalyst according to claim 2, characterised in that the copper content of the catalyst is from about 7 to about 9 weight percent.

4. A process for preparing ethylene dichloride by catalytic oxychlorination of ethylene characterised in that the catalyst is cupric chloride and potassium chloride deposited on support material wherein substantially all of the cupric chloride and potassium chloride is present as the trichlorocuprate complex $KCuCl_3$.

5. A process according to claim 4, characterised in that the oxychlorination is effected with hydrogen chloride and oxygen using a mole ratio of ethylene: hydrogen chloride:oxygen of 1.0:1.5:0.5 at a temperature of 150°C to 500°C for 10 seconds to about 2 minutes.

6. A process according to claim 4 or 5, characterised in that the oxychlorination is effected in a fluid bed and that the particle size of the catalyst is 30 to 200 mesh (U.S. Sieve).

7. A process according to claim 6, characterised in that the support material is attapulgite clay.

8. A process according to any of claims 4 to 7, characterised in that the copper content of the catalyst is from about 4 to about 12 weight percent.

9. A process according to claim 8, characterised in that the copper content of the catalyst is from about 7 to about 9 weight percent.

10. A process for preparing a catalyst suitable for use in the oxychlorination of ethylene characterised in that a support material is treated with an aqueous solution consisting essentially of equimolar quantities of cupric chloride and potassium chloride and the treated support material is dried, the treating and drying steps being conducted at a temperature in excess of 76°C.

# EUROPEAN SEARCH REPORT

Application number

EP 81 30 2164

## DOCUMENTS CONSIDERED TO BE RELEVANT

| Category | Citation of document with indication, where appropriate, of relevant passages | Relevant to claim |
|---|---|---|
| | <u>GB - A - 1 277 510</u> (ESSO) <br><br> * page 1, lines 12-23; page 2, lines 14-95; claims 1-23 * | 1 |
| | <u>US - A - 3 691 098</u> (B. CALCAGNO) <br><br> * abstract; column 1, lines 6-40; column 2, lines 9-59; claims 1-12 * | 1-3,5, 6,8,9 |
| D | <u>FR - A - 2 409 793</u> (PPG IND.) <br><br> * page 1, line 30 - page 2, line 24; page 4, lines 17-36; claims 1-20 * <br><br> & US - A - 4 172 052 | 1-7 |
| A | <u>GB - A - 2 003 878</u> (VULCAN MET. CO) | |
| A | <u>GB - A - 997 825</u> (I.C.I.) | |

CLASSIFICATION OF THE APPLICATION (Int. Cl.³)

B 01 J 27/10
C 07 C 17/156
         19/045

TECHNICAL FIELDS SEARCHED (Int. Cl.³)

B 01 J 27/10
         27/08
C 07 C 17/156
         19/00
         19/02
         19/045

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 03-09-1981 | LO CONTE |

EPO Form 1503.1   06.78